# NEUE EUROPÄISCHE PATENTSCHRIFT

(11) **EP 3 906 982 B2**
(45) Veröffentlichungstag und Bekanntmachung des Hinweises auf die Entscheidung über den Einspruch: **13.08.2025**
(45) Hinweis auf die Patenterteilung: 16.03.2022
(21) Anmeldenummer: 20173766.5
(22) Anmeldetag: 08.05.2020
(51) Int. Cl.: B01D 15/36, B01J 20/28, B01J 41/20, B01D 15/12, B01J 20/32, C12N 15/10

(54) **VERFAHREN ZUR PLASMIDREINIGUNG UNTER GLEICHZEITIGER ABREICHERUNG VON ENDOTOXINEN**
PLASMA CLEANING METHOD WITH SIMULTANEOUS DEPLETION OF ENDOTOXINS
PROCÉDÉ DE NETTOYAGE DE PLASMIDE À APPAUVRISSEMENT SIMULTANÉ D'ENDOTOXINES

(43) Veröffentlichungstag der Anmeldung: 10.11.2021
(73) Patentinhaber: AXAGARIUS GmbH & Co. KG, 52355 Düren (DE)
(72) Erfinder: MEUSEL, Markus, 52146 Würselen (DE)
(74) Vertreter: dompatent von Kreisler Selting Werner - Partnerschaft von Patent- und Rechtsanwälten mbB

(56) Entgegenhaltungen:
- EP-A1- 1 125 943
- WO-A1-93/11218
- WO-A2-2004/060277
- DE-A1- 10 201 858
- DE-B4- 19 859 703
- DE-U1- 202005 010 007
- ALEKSEI ROZKOV ET AL: "Large-scale production of endotoxin-free plasmids for transient expression in mammalian cell culture", BIOTECHNOLOGY AND BIOENGINEERING, vol. 99, no. 3, 15 February 2008 (2008-02-15), US, pages 557 - 566, XP055734310, ISSN: 0006-3592, DOI: 10.1002/bit.21603
- RUI MA ET AL: "Removing endotoxin from plasmid samples by Triton X-114 isothermal extraction", ANALYTICAL BIOCHEMISTRY, ACADEMIC PRESS, AMSTERDAM, NL, vol. 424, no. 2, 10 February 2012 (2012-02-10), pages 124 - 126, XP028406546, ISSN: 0003-2697, [retrieved on 20120225], DOI: 10.1016/J.AB.2012.02.015
- ANONYMOUS: "PureLink® HiPure Plasmid Filter Purification Kits Handbuch", INVITROGEN BY LIFE TECHNOLOGIES, 2011, pages 1 - 36, XP093014354
- NucleoBond® PC EF Kits Handbuch, veröffentlicht 2016
- NucleoBond® Xtra EF Kits Handbuch, veröffentlicht Januar 2020
- EndoFree Plasmid Purification Handbuch, veröffentlicht 2015

## Beschreibung

Die vorliegende Erfindung betrifft ein Verfahren zur Abtrennung von Endotoxinen bei der Plasmidreinigung unter Verwendung eines mit Detergens beladenen zylindrischen Tiefenfilters. Weiterhin wird ein mit Detergens beladener zylindrischer Tiefenfilter, eine Trennvorrichtung und Kit mit solch einem Tiefenfilter, die für das erfindungsgemäße Verfahren geeignet sind, zur Verfügung gestellt.

### Hintergrund der Erfindung

Endotoxine sind Lipopolysaccharide (LPS) aus der Zellwand Gram-negativer Bakterien, inklusive des gentechnologisch und biotechnologisch wichtigen Bakteriums *Escherichia coli.* Letzteres Bakterium wird sehr häufig in biotechnologischen Verfahren eingesetzt, so dass Endotoxine sehr relevante Kontaminationen bei biotechnologisch hergestellten Produkten sind. Endotoxine gehören zu den Pyrogenen, das heißt, sie können bei Kontakt mit Schleimhäuten und bei Übertritt ins Blut bei Menschen und manchen Tierarten Fieber erzeugen. Außerdem aktivieren sie eine Reihe von Signalwegen von immunkompetenten Zellen, die entweder zu einer Entzündung oder zu einem programmierten Zelltod (Apoptose) dieser Zellen führen können. Sie sind schon in niedrigsten Konzentrationen (unterer pg/ml-Bereich) biologisch wirksam. Insbesondere bei der Transfektion, d.h. dem Einbringen von Fremd-Nukleinsäuren in eukaryontische Zellen, wird die Transfektionseffizienz durch vorhandene Endotoxine erheblich reduziert. Insbesondere bei gentherapeutischen Ansätzen muss die Konzentration von Endotoxinen daher auf ein Minimum reduziert werden.

Die sogenannte "transfection-grade" Qualität mit sehr niedrigen Konzentrationen an Endotoxinen bei der Plasmidreinigung wird üblicherweise mit Anionenaustauschern erreicht. Die Bakterien werden lysiert (sogenannte alkalische Lyse), die Plasmide binden an eine Anionenaustauschermatrix und werden unter hochsalz-Bedingungen eluiert. Letztlich werden die Nukleinsäuren meist durch eine Alkoholfällung konzentriert und entsalzt. Die Reinigung findet meist im s.g. Midi- oder Maxi-Format mit vorgefertigten Anionenaustauschersäulen statt. Es gibt auch Methoden der Plasmidreinigung, die sogenannten Minipreps, bei denen noch vorhandene Endotoxine die nachfolgende Analytik oder Verwendung der Plasmide nicht oder kaum stört. Bei diesen Verfahren werden häufig Silicamembranen zur Bindung der Plasmide eingesetzt.

Endotoxine werden beim Aufschluss von bakteriellen Zellen (der Lyse) aus der Zellmembran freigesetzt und können nur schwer von Nukleinsäuren abgetrennt werden. Durch eine ebenfalls negative Nettoladung und die Größe der Endotoxine, die mizellare Strukturen bilden, erfolgt eine Verschleppung in die Fraktionen der aufgereinigten DNA, insbesondere von gereinigter Plasmid DNA. Endotoxingehalte bei der Aufreinigung von DNA werden üblicherweise in Endotoxin Units (EU) pro µg DNA angegeben.

Der Entfernung oder Reduzierung von Endotoxinen in Plasmid- oder sonstigen DNA-Präparationen kommt somit eine große wirtschaftliche Bedeutung zu. Die Entfernung der Endotoxine bei der Plasmidreinigung ist ein bekanntes Problem des Standes-der-Technik.

Bei allen Verfahren zur Plasmidreinigung muss das Lysat vor der Reinigung der Plasmide von den unlöslichen Zellbestandteilen getrennt werden. Nach dem Stand-der-Technik werden Plasmide aus Bakterien durch die s.g. alkalische Lyse aufgeschlossen. Dabei werden die Zellen zunächst in einem Resuspensionspuffer suspendiert. Die suspendierten Zellen werden durch Zugabe von Natriumhydroxid (NaOH) und dem Detergens SDS (Natriumdodecylsulfat) lysiert. Die alkalischen Bedingungen zusammen mit dem Detergens führen zu einer nahezu vollständigen Denaturierung aller Zellbestandteile. Auch die vorhandene doppelsträngige genomische DNA und die Plasmid-DNA werden denaturiert. Anschließend wird die Lösung durch Zugabe von Kaliumacetat neutralisiert. Hierbei fällt das unlösliche Kaliumdodecylsulfat aus, gleichzeitig wird ein Großteil der Zellkomponenten und Zellwand ebenfalls ausgefällt. Der pH-Wertanstieg durch die Neutralisation führt zu einer Renaturierung der Plasmid-DNA, nicht aber der genomischen DNA, die gemeinsam mit den übrigen Zellbestandteilen ausgefallen bleibt.

Werden nun die unlöslichen Bestandteile von den löslichen Bestandteilen mit den Plasmiden getrennt, können auf sehr elegante Weise genomische DNA und zelluläre Verunreinigungen entfernt werden. Wie eingangs besprochen, verbleibt allerdings ein Teil der Endotoxine als gelöste Kontamination in der Plasmidfraktion. Die Trennung der ausgefällten, unlöslichen Zellbestandteile vom löslichen Teil mit den Plasmiden kann auf verschiedenen Wegen erfolgen. Ein einfacher Weg ist die Trennung mittels Zentrifugation. So kann der flüssige Teil der Probe vom Pellet abgetrennt werden. Zentrifugation ist allerdings apparativ aufwendig und beim Transfer der flüssigen Probe kann auch leicht ein Teil des Präzipitates mit in die Probe gelangen und diese kontaminieren.

Alternativ bietet sich die Filtration über Faltenfilter an, bei denen die unlöslichen Bestandteile im Filter zurückgehalten werden. Hier ist keine Zentrifuge nötig, allerdings läuft die Flüssigkeit nur sehr langsam durch den Filter, der zudem leicht verstopfen kann. Dies führt zu Zeitverlusten.

Letztlich ist noch die Druckfiltration beschrieben, bei der das Lysat ein eine Spritze eingefüllt und durch einen geeigneten Filter gepresst wird. Je nach Ausführung ist hier aber eine Vorinkubation in der Spritze notwendig, zudem besteht die Gefahr des Verstopfens des Filters. Auch muss die Zellmenge sehr genau eingestellt werden, um Überladungen und Verstopfen zu vermeiden.

In der DE 10201858A1 ist ein zylindrischer Filter beschrieben, der vorzugsweise direkt in eine geeignete Trennvorrichtung zur Plasmidreinigung eingesteckt ist. Das Lysat wird hierbei in den Filter eingegeben, Zelltrümmer und andere unlösliche Bestandteile werden zurückgehalten und das klare Filtrat mit den Plasmiden fließt vom Filter direkt in den Trennvorrichtung, die beispielsweise als Anionenaustauscher ausgebildet sein kann. Der Filter wird bevorzugt als Papierfilter ausgestaltet, der eng an der Wandung und am Boden der Trennvorrichtung anliegt. Nachteilig ist hier die Gefahr der Verstopfung des Filters, insb. wenn dieser in direkten Kontakt mit der Wandung der Trennvorrichtung kommt.

Eine vergleichbare Filtereinrichtung wird in der DE 202005010007U1 beschrieben. Hier ist das Filterelement allerdings als Tiefenfilter ausgebildet. Der Filter, z.B. gefertigt aus Cellulosefasern hat Eigenstabilität, eine strukturierte Oberfläche und verringert die Verstopfungsgefahr durch die Tiefenfilter-Wirkung erheblich.

Für die Entfernung der Endotoxine wurden viele Verfahren beschrieben. So können Endotoxine beispielsweise an Polymyxin B Affinitätsmedien gebunden und aus der Probe entfernt werden. Am häufigsten werden nach dem Stand-der-Technik nichtionische Detergenzien eingesetzt, die die Endotoxine komplexieren und Mizellen bilden, die vom Rest der biologischen Probe abgetrennt werden können. Das am häufigsten hierzu eingesetzte Detergens ist Triton^{®} X-114. Dabei handelt es sich um ein nicht-ionisches Detergens (CAS Nr. 9036-19-5, Polyethylenglycol-[4-(1,1,3,3-tetramethylbutyl)phenyl]ether mit 7 bis 8 Ethylenglycoleinheiten; Synonym: (1,1,3,3-Tetramethylbutyl)phenyl-polyethylene glycol).

US 6,194,562 B1 beschreibt ein Verfahren, bei dem Endotoxine aus der aufgeschlossenen biologischen Probe von Nukleinsäuren durch Bindung an eine Silicamatrix abgetrennt werden. Die Bindebedingungen werden dabei so eingestellt, dass nur die Endotoxine selektiv an die Matrix binden, die Nukleinsäuren passieren diese Matrix und können anschließend weiter aufgereinigt werden.

Alternativ zur selektiven Anbindung können Endotoxine auch selektiv von einer festen Phase abgewaschen werden. In der DE 10 2016 106 271 B4 wird ein Verfahren beschrieben, bei dem Nukleinsäuren in einem ersten Schritt an eine feste Matrix gebunden werden. Der Teil der Endotoxine, der ebenfalls unter diesen Bedingungen gebunden wird, wird anschließend durch eine Waschlösung, die Amin-Verbindungen und ein organisches Lösungsmittel enthält, von der festen Matrix entfernt. Die gebundenen Nukleinsäuren werden anschließend weiter aufgereinigt.

Speziell für die Plasmidaufreinigung mittels Anionenaustauschern sind dem Stand-der-Technik nach zwei Verfahren bekannt, die zu einer deutlichen Reduzierung der Endotoxine führen.

In der EP 0743949B1 wird das Detergens Triton^{®} X-114 verwendet. Die Bakterien werden in einem dem Stand-der-Technik nach bekannten Verfahren alkalisch lysiert und anschließend mit einem Triton^{®} X-114-haltigen *Endotoxin Removal Buffer* (ERB) für 30 min auf Eis inkubiert. Während dieser Inkubation werden die Endotoxine zusammen mit dem Detergens komplexiert und es bilden sich Mizellen. Wird dieses Gemisch anschließend auf einen Anionenaustauscher gegeben, so wird ein Großteil der Endotoxine nicht gebunden und passiert die feste Matrix. Die Plasmide im Lysat werden unter den gewählten Bedingungen hingegen an den Anionenaustauscher gebunden. Verbleibende Endotoxine werden dann mit einem Detergens-haltigen Waschpuffer noch weiter reduziert, bevor die Plasmide von der Matrix eluiert werden. Nachteilig bei diesem Verfahren ist die zusätzliche, langwierige Inkubation auf Eis mit einem zusätzlichen Puffer. Das Verfahren wird damit aufwändiger und länger in der Durchführung.

In der EP 1125943B1 wird ein Verfahren beschrieben, dass auf die Vorinkubation mit dem *Endotoxin Removal Buffer* verzichtet. Um die Bindebedingungen am Anionenaustauscher einzustellen (andernfalls binden die Plasmide nicht) werden diese gemäß dem Stand der Technik mit einem geeigneten Puffer äquilibriert. In dem in der EP 1125943B1 beschriebenen Verfahren wird das Detergens Triton^{®} X-114 zusammen mit dem Äquilibrierungspuffer auf den Anionenaustauscher aufgebracht. Wird nun die biologische Probe nach der Lyse und der Klärung des Lysates auf den Anionenaustauscher gegeben, so verhindert das Detergens die Anbindung der Endotoxine, die mit dem Rest der Probe ohne Bindung die Säule passieren. Wie bei der EP 0 743 949 werden die Plasmide im Lysat unter den gewählten Bedingungen an den Anionenaustauscher gebunden und verbleibende Endotoxine mit einem Detergens-haltigen Waschpuffer noch weiter reduziert, bevor die Plasmide von der Matrix eluiert werden. Nachteilig bei diesem Verfahren ist die zusätzliche Äquilibrierung des Anionenaustauschers mit dem Detergens-haltigen Puffer.

Für kommerzielle Produkte nach den beiden oben genannten Verfahren werden in sogenannten EF- (Endotoxin-free) oder "transfection grade" Produkten Endotoxingehalte unter 0,1 EU/µg DNA erzielt.

Bei der Herstellung von kommerziellen Kits für die Nukleinsäurereinigung spielt die Stabilität und Lagerfähigkeit der Komponenten eine wichtige Rolle. Daher wird bereits jetzt eine Reihe von Komponenten durch Gefriertrocknung stabilisiert. Die Gefriertrocknung von einzelnen Komponenten wie Enzymen bietet verlängerte Lebensdauer, vereinfacht das Handling und den Gebrauch und reduziert das Risiko einer Kontamination. Bezieht man komplexe Lösungen in den Gefriertrocknungsprozess mit ein, z.B. Puffer, die als Einzelreagenz oftmals in großen Volumina in einem Kit vorliegen, so können potentiell auch Gewicht und Volumen reduziert werden, was Transportkosten minimiert und einen wichtigen Umweltbeitrag liefert. Gerade der Transport ist weniger kritisch als der von Flüssigreagenzien und erleichtert oder verhindert spezielle Anforderungen und Kennzeichnungen an den Gefahrguttransport.

Gerade bei den bereits erwähnten Midi- oder Maxi-Plasmidpräparationen werden große Flüssigkeitsvolumina benötigt und der Einsatz gefriergetrockneter Reagenzien, die vom Anwender lediglich durch Wasserzugabe rekonstituiert werden, würde viele Vorteile bieten. Während einige Puffer relativ leicht als Lyophilisat bereitgestellt werden können, ist dies bei Triton^{®} X-114 haltigen Puffern nicht möglich, da das Detergens nicht als Pulver, sondern nur als viskose Flüssigkeit vorliegt.

### Kurzbeschreibung der Erfindung

In der vorliegenden Erfindung wurde überraschenderweise festgestellt, dass ein Tiefenfilter für die Lysatklärung bei der Plasmidreinigung so behandelt werden kann, dass die Endotoxine in der nachfolgenden Anionenaustauscherreinigung zu einem hohen Grad reduziert werden können. Die Erfindung betrifft somit folgende Aspekte:
(1) Ein Verfahren zur Plasmidreinigung unter gleichzeitiger Abreicherung von Endotoxinen umfassend die Lysatklärung eines Plasmid-haltigen Zelllysates in einem mit nicht-ionischem Detergens beladenen zylindrischem Tiefenfilter, das direkte Überführen des Filtrats mit dem geklärten Zelllysat auf einen fluidisch nachgeschalteten Anionenaustauscher, und die Elution des gereinigten und Endotoxinabgereicherten Plasmidmaterials von dem Anionenaustauscher durch einen Elutionspuffer; wobei der mit nicht-ionischen Detergens beladene Tiefenfilter mit dem nicht-ionischen Detergens imprägniert ist oder das nicht-ionische Detergens bei der Tiefenfilterherstellung in das Fasergeflecht eingebracht wird, und wobei der zylindrische mit Detergens beladene Tiefenfilter und der Anionenaustauscher in einer Trennvorrichtung vorliegen, die ein säulenartiges Außengefäß mit einer oben-seitigen Einfüllöffnung und mit dem darin eingesetzten zylindrischen Tiefenfilter, der eine Filterhülse mit einem Filterboden und einem davon hochgehenden Filtermantel ist, wenigstens eine poröse Trägerschicht und eine auf dieser Trägerschicht befindliche Anionenaustauscherschicht umfasst;
(2) ein mit nicht-ionischen Detergens beladener zylindrischer Tiefenfilter wie in (1) definiert;
(3) eine Trennvorrichtung wie in (1) definiert mit einem wie in (1) definiert, mit nicht-ionischen Detergens beladenen, zylindrischen Tiefenfilter; und
(4) einen Kit zur Plasmidreinigung unter gleichzeitiger Abreicherung von Endotoxinen umfassend wenigstens einen mit nicht-ionischen Detergens beladenen zylindrischen Tiefenfilter von Aspekt (2) und/oder wenigstens eine Trennvorrichtung von Aspekt (3).

Der/die vorstehend genannte Tiefenfilter, Trennvorrichtung und Kit sind dabei für Plasmidreinigungsverfahren von Aspekt (1) geeignet.

Erfindungsgemäß werden bevorzugt mit dem Detergens Triton^{®} X-114 imprägnierte Cellulosefilter für die Lysatklärung eingesetzt. Bevorzugt wird der Filter fabrikationsseitig mit dem nicht-ionischen Detergens imprägniert, oder das nicht-ionische Detergens wird bereits während der Filterherstellung in die Cellulosematrix eingebracht. Dieser Filter wird dann in eine bevorzugt zylindrische Trennvorrichtung mit einem Anionenaustauscher eingebracht. Der Verzicht auf Triton^{®} X-114 in den üblichen Puffern nach dem Stand-der-Technik erlaubt zudem eine Gefriertrocknung der Puffer, mit den oben genannten Vorteilen, ohne auf die Verwendung des nicht-ionischen Detergens zur Endotoxinentfernung zu verzichten.

Nach alkalischer Lyse wird das Zelllysat in den Filter gegeben. Die Tiefenfilter-Wirkung verhindert ein Verstopfen des Filters. Vorteilhaft ist eine fabrikationsseitig raue, unebene Außenseite des selbsttragenden Filters, so dass dieser nicht an der Innenwand der Trennvorrichtung, in die er eingesteckt ist, anhaftet. Das Ablaufen des Filtrats wird somit erleichtert und die Trennleistung verbessert.

Bei Kontakt mit der lysierten Probe wird der erfindungsgemäße Filter benetzt. Das nicht-ionische Detergens löst sich dabei vom Filter und vermischt sich mit dem Filtrat. Auf diese Weise wird das Filtrat ohne weitere Schritte oder zusätzliche Puffer oder Reagenzien mit dem Detergens versetzt. Trifft das Gemisch aus filtrierter biologischer Probe und zugesetztem nicht-ionischen Detergens auf den Anionenaustauscher, so unterbindet das Detergens die Anbindung der Endotoxine. Die erhaltene Plasmidpräparation hat insofern einen deutlich niedrigeren Gehalt an Endotoxinen, als eine vergleichbare Präparation mit nicht imprägnierten Filtern und unbehandelten Anionenaustauschern.

Alternativ kann auch über den in die Trennvorrichtung eingesteckten mit nicht-ionischen Detergens imprägnierten Tiefenfilter ein Puffer zur Äquilibrierung des nachgeschalteten Anionenaustauschers eingebracht werden.

Überraschenderweise reicht das Inkontaktbringen der Probe mit dem erfindungsgemäßen Filter aus, um genügend nicht-ionisches Detergens freizusetzen, um die Anbindung von Endotoxinen deutlich zu reduzieren. Das Verfahren umgeht daher die bekannten Nachteile aus dem Stand-der-Technik, wie etwa eine zusätzliche Inkubation mit speziellen Puffern auf Eis oder die Vorab-Inkubation/Benetzung des Anionenaustauschers mit dem Detergens. Das mit dem erfindungsgemäßen Verfahren erhaltene Eluat kann, bedingt durch die Abwesenheit von Detergens im Elutionspuffer, sehr gut weiterverarbeitet werden, da es im wesentlichen Detergens-frei ist.

### Kurzbeschreibung der Figuren

Figur 1: Trennvorrichtung mit Anionenaustauscher und eingestecktem erfindungsgemäßem Filter. 1, Cellulosefilterhülse; 2, Einlassöffnung des Filters; 3, Öffnung der Trennvorrichtung (Säulenkörper); 4, Trennvorrichtung (Säulenkörper); 5, Polyethylenfritten zum Rückhalt des Anionenaustauschermaterials; 6, Anionenaustauscher (hier als Pulverschicht zwischen zwei Polyethylenfritten); 7, Auslassöffnung der Trennvorrichtung.
Figur 2: DNA-Ausbeute in Abhängigkeit von der Menge an Triton^{®} X-114 gemäß Ausführungsbeispiel 1. Ansatz G: Kommerzieller Kit zur Plasmidreinigung mit Endotoxin-Abreicherung.
Figur 3: Endotoxingehalt der isolierten Plasmid-DNA in Abhängigkeit von der Menge an Triton^{®} X-114 gemäß Ausführungsbeispiel 1. Ansatz G: Kommerzieller Kit zur Plasmidreinigung mit Endotoxin-Abreicherung.
Figur 4: DNA-Ausbeute in Abhängigkeit von der Menge an Triton^{®} X-114 gemäß Ausführungsbeispiel 2. Ansatz G: Kommerzielles Produkt zur Plasmidreinigung ohne besondere Maßnahmen zur Endotoxinabreicherung.
Figur 5: Endotoxingehalt der isolierten Plasmid-DNA in Abhängigkeit von der Menge an Triton^{®} X-114 gemäß Ausführungsbeispiel 2. Ansatz G: Kommerzielles Produkt zur Plasmidreinigung ohne besondere Maßnahmen zur Endotoxinabreicherung.
Figur 6: Endotoxingehalt der isolierten Plasmid-DNA in Abhängigkeit von der Menge an Triton^{®} X-114 gemäß Ausführungsbeispiel 2. Endotoxingehalt hier auf Kontrolle ohne Benetzung als 100% normiert. Ansatz G: Kommerzielles Produkt zur Plasmidreinigung ohne besondere Maßnahmen zur Endotoxinabreicherung.
Figur 7: DNA-Ausbeute. Versuchsdurchführung gemäß Ausführungsbeispiel 3. Ansatz A: Kontrolle mit nicht imprägnierter Filterhülse. Ansatz B: Filterhülse mit Triton^{®} X-114, keine Benetzung während der Äquilibrierung. Ansatz C: Filterhülse mit Triton^{®} X-114, Benetzung während der Äquilibrierung.
Figur 8: Endotoxingehalt der isolierten Plasmid-DNA. Versuchsdurchführung gemäß Ausführungsbeispiel 3. Ansatz A: Kontrolle mit nicht imprägnierter Filterhülse. Ansatz B: Filterhülse mit Triton^{®} X-114, keine Benetzung während der Äquilibrierung. Ansatz C: Filterhülse mit Triton^{®} X-114, Benetzung während der Äquilibrierung.

### Detaillierte Beschreibung der Erfindung

Das Verfahren gemäß Aspekt (1) der Erfindung umfasst vorzugsweise die folgenden Schritte:
(a) Lyse des Zellmaterials,
(b) Überführen des Zelllysates in dem mit nicht-ionischen Detergens beladenen zylindrischen Tiefenfilter,
(c) direktes Überführen des Filtrats mit dem geklärten Zelllysat auf den fluidisch nachgeschalteten Anionenaustauscher,
(d) Waschen des Tiefenfilters und des fluidisch nachgeschalteten Anionenaustauschers mit einem Wasch-/Äquilibrierungspuffer, und
(e) Elution des gereinigten Plasmidmaterials von dem Anionenaustauscher durch einen Elutionspuffer.

Dabei kann der vorstehende Waschschritt (d) zwei separate Waschschritte (d1) und (d2) umfassen, nämlich einen Schritt (d1) das Waschen des Tiefenfilters und des fluidisch nachgeschalteten Anionenaustauschers mit einem ersten Wasch-/Äquilibrierungspuffer und (d2) direktes Waschen des Anionenaustauschers mit einem zweiten Waschpuffer. Der erste Wasch-/Äquilibrierungspuffer und der zweite Waschpuffer können dabei gleich oder unterschiedlich sein und die nachfolgend angegebenen Bestandteile aufweisen.

Der in dem erfindungsgemäßen Verfahren verwendete zylindrische Tiefenfilter weist ein regeloses Fasergeflecht, welches aus Cellulose-, Glas-, Kunststoff- und Metallfasern und Gemischen derselben besteht, wobei ein regeloses Fasergeflecht aus Cellulosefasern besonders bevorzugt ist, und/oder Eigenstabilität auf. Solch ein besonders bevorzugter Tiefenfilter wird aus einer Cellulosesuspension durch Absaugen mit einer Saugform erhalten, wobei die Saugform den Innendurchmesser und die Geometrie des Filters vorgibt. Über die Konzentration der suspendierten Cellulose, den Unterdruck und die Zeit kann die Dicke und Textur des Filters beeinflusst werden. Besonders vorteilhaft ist es, wenn die Filteraußenseite so erstellt wird, dass eine bewusst raue und unebene Oberfläche erhalten wird. Dies verbessert die Filterleistung beim Einstecken in eine Trennvorrichtung, da der Filter dann weniger an der Oberfläche der Trennvorrichtung anhaftet. Solche Tiefenfilter sind dem Stand-der-Technik nach auch als sogenannte Extraktionshülsen bekannt.

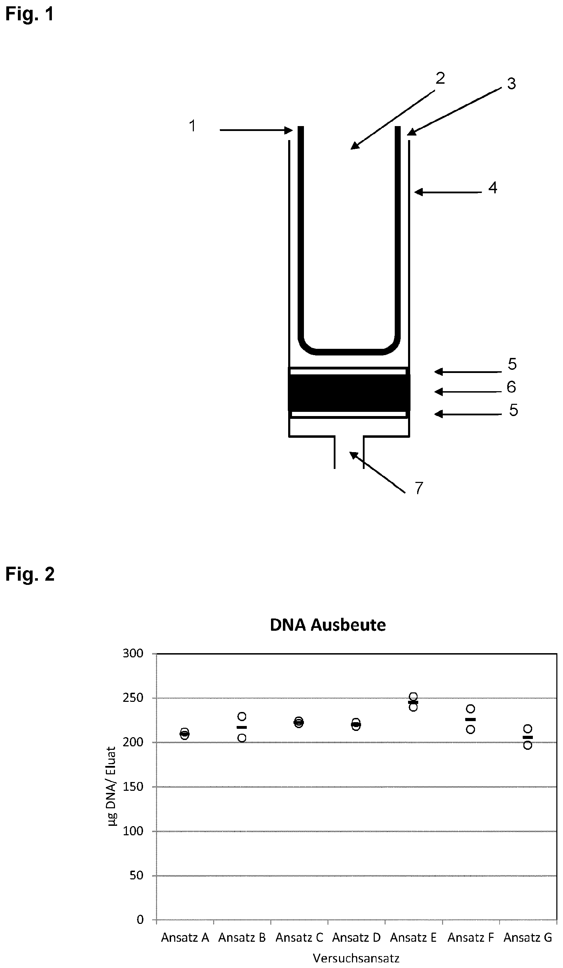

Eine bevorzugte Ausführungsform des Verfahrens gemäßen Aspekt (1) der Erfindung ist in der Figur 1 gezeigt. Dabei liegen der zylindrische Tiefenfilter 1 und der Anionenaustauscher in einer Trennvorrichtung vor, die ein säulenartiges Außengefäß 4 mit einer obenseitigen Einfüllöffnung 3 und mit einem darin eingesetzten zylindrischem Tiefenfilter 1, der einen Filterboden und einen davon hochgehenden Filtermantel aufweist, wenigstens eine poröse Trägerschicht 5 und eine auf dieser Trägerschicht 5 befindliche Anionenaustauscherschicht 6 umfasst. Oberhalb der Anionenaustauscherschicht kann sich eine weitere Trägerschicht 5 befinden, die das Anionenaustauschermaterial fixiert und somit eine gleichmäßige Oberfläche der Anionenaustauscherschicht 6 in der Trennvorrichtung gewährleistet. Der Tiefenfilter 1 (d. h. die Filterhülse) ist zylindrisch ausgeformt und wird in eine zylindrische Trennvorrichtung eingesteckt.

Der Filter besteht bevorzugt aus Cellulose und hat in den hier gezeigten Ausführungsbeispielen eine Länge von 9 cm, einen Durchmesser von 18 mm und eine Wandstärke von ca. 2-3 mm. Die verwendeten Filter haben zudem an der Einlassöffnung einen Kragen ausgebildet, der den Filter im oberen Teil der Trennvorrichtung fixiert. Erfindungsgemäß sind aber auch alle Abwandlungen in Geometrie, Längen- und Dickenmaßen etc. möglich.

Die Imprägnierung der Filter mit dem nicht-ionischen Detergens kann erfindungsgemäß in verschiedenen Verfahren erfolgen. Zum einen kann das nicht-ionische Detergens bevorzugt fabrikationsseitig bei der Herstellung der Filter eingebracht werden. Hierbei kann das nicht-ionische Detergens der Cellulosesuspension während der Herstellung zugesetzt werden. Beim Trocknen des Filters verbleibt das nicht-ionische Detergens dann auf dem Filter. Alternativ kann der fertige Filter mit einer konzentrierten nicht-ionischen Detergenslösung imprägniert und anschließend getrocknet werden.

In der Literatur und insb. der Patentliteratur werden viele verschiedene Detergenzien im Kontext der Endotoxinentfernung beschrieben. Für das erfindungsgemäße Verfahren geeignete nicht-ionische Detergenzien, sind vorzugsweise Polyethylenglyco144-(1,1,3,3-tetramethylbuty1)-phenynether wie Polyethylenglyco144-(1,1,3,3-tetramethylbutyl)phenynether mit 7-8 oder 9-10 Ethylenglycoleinheiten (Triton^{®} X-114 bzw. Triton^{®} X-100). Das nicht-ionische Detergens mit dem größten Potential ist das nicht-ionische Tensid Triton^{®} X-114, welches in den nachfolgenden Ausführungsbeispielen eingesetzt wird.

In dem erfindungsgemäßen Verfahren nach Aspekt (1) besteht der Anionenaustauscher bevorzugt aus funktionalisierten Silicapartikeln wobei mit Methylaminoethanol oder Diethylaminoethanol funktionalisierte Silicapartikel besonders bevorzugt sind.

In dem erfindungsgemäßen Verfahren erfolgt die Lyse des Zellmaterials bevorzugt durch Versetzen mit einem alkalischen Lysepuffer und nachfolgender Neutralisation mittels eines Neutralisationspuffers, wobei diese Puffer vorzugsweise frei von nicht-ionischen Detergenzien sind.

In einer bevorzugten Ausführungsform des erfindungsgemäßen Verfahrens sind der Elutionspuffer und/oder der Wasch/Äquilibrierungspuffer Detergens-frei.

In einer anderen bevorzugten Ausführungsform des erfindungsgemäßen Verfahrens enthält der Wasch/Äquilibrierungspuffer Tris, Ethanol, ein für den verwendeten Anionenaustauscher geeignetes Anion (wie z.B. Chlorid) und ein nicht-ionisches Detergens. In solchen Ausführungsformen, in denen der Anionenaustauscher nicht gemeinsam mit dem Tiefenfilter in einer Trennvorrichtung vorliegt, die nicht Gegenstand der Erfindung sind, nicht nur ein Polyethylen-glyco144-(1,1,3,3-tetramethylbutyl)phenyether wie Triton^{®}X-114 und Triton^{®} X-100, sondern auch ein ethoxylierter Sorbitanfettsäureester wie Tween^{®} 20 (Polyoxyethylen(20)-sorbitanmonolaurat) oder ein anderes nichtionisches De- tergens (wie Alkoholethoxylate, oder Nonylphenolethoxylate) sein. In den erfindungsgemäßen Verfahrens, liegt der Anionenaustauscher gemeinsam in dem Tiefenfilter in einer Trennvorrichtung vor, dabei ist andererseits bevorzugt, dass zusätzliches nicht-ionisches Detergens in dem Wasch/Äquilibrierungspuffer dasselbe nicht-ionische Detergens ist, dass in dem imprägniertem Tiefenfilter vorhanden ist.

In einer weiteren bevorzugten Ausführungsform des erfindungsgemäßen Verfahrens steht das Volumen des Wasch/Äquilibrierungspuffers im Verhältnis von 10 bis 20 zum Totvolumen des Anionenaustauschers. "Totvolumen" hat dabei die für den Fachmann geläufige Bedeutung, nämlich die für das Auffüllen des trockenen Ionenaustauschermaterials erforderliche Flüssigkeitsmenge/-volumen. In einer weiteren bevorzugten Ausführungsform des erfindungsgemäßen Verfahrens enthält der Elutionspuffer Salze mit sterisch kleine Anionen als Gegenionen für den Anionenaustauscher wie Chlorid(C11-, Jodat(IO3)-, Borat(B0₃³⁻), Fluorid(F⁻) und Thiocyanat(SCNilonen, wobei Salze mit Chloridionen besonders bevorzugt sind. Kationen der Salze können Alkali- und Erdalkalimetalle wie z.B. Kalium, Natrium, Magnesium oder Calcium sein.

In einer weiteren bevorzugten Ausführungsform des erfindungsgemäßen Verfahrens wird die Elution durch einen pH-Shift in den alkalischen pH Bereich in Bezug auf den pH des Wasch/Äquilibrierungspuffers unterstützt. Dies bedeutet, dass der pH des Elutionspuffers im Bereich von pH 8 bis 10 liegt, wohingegen der pH des Wasch/Äquilibrierungspuffers üblicherweise im Bereich von 6 bis 7 liegt. In einer weiteren bevorzugten Ausführungsform des erfindungsgemäßen Verfahrens steht das Volumen des Elutionspuffers im Verhältnis von 5 bis 20 zum Totvolumen des Anionen-austauschers. Besonders bevorzugt ist ein etwa 10-faches des Totvolumens.

Aspekt (2) der Erfindung betrifft den mit nicht-ionischen Detergens beladenen zylindrischen Tiefenfilter von Aspekt (1). Bezüglich bevorzugter Ausführungsformen des Tiefenfilters und dessen Herstellung wird auf die entsprechenden Aus-führungen im Zusammenhang mit Aspekt (1) verwiesen.

Aspekt (3) der Erfindung betrifft eine Trennvorrichtung von Aspekt (1) mit einem wie in (1) definierten, mit nicht-ionischen Detergens beladenen, zylindrischen Tiefenfilter. Bezüglich bevorzugter Ausführungsformen der Trennvorrichtung wird auf die entsprechenden Ausführungen im Zusammenhang mit dem Plasmidreinigungsverfahren von Aspekt (1) verwiesen.

Aspekt (4) der Erfindung betrifft einen Kit zur Plasmidreinigung unter gleichzeitiger Abreicherung von Endotoxinen umfassend wenigstens einen mit nicht-ionischen Detergens beladenen zylindrischen Tiefenfilter von Aspekt (2) und/oder wenigstens eine Trennvorrichtung von Aspekt (3). Der Kit kann weiterhin einen oder mehrere geeignete Lysepuffer, Neutralisationspuffer, Wasch-/Äquilibrierungspuffer und Elutionspuffer, insbesondere wie im Vorstehenden beschrieben enthalten. Bevorzugt ist dabei, dass einer oder mehrere der genannten Puffer Detergens-frei sind, als Lyophilisate (Pufferkonzentrate) in dem Kit vorliegen und durch Zugabe von Wasser gebrauchsfertig gemacht werden können. Besonders bevorzugt ist, dass alle benötigten Puffer Detergens-frei sind und als Lyophilisate bereitgestellt werden, was bedeutet, dass dann das für die Abreicherung von Endotoxinen benötigte nicht-ionische Detergens ausschließlich über die imprägnierten Tiefenfilter bereitgestellt wird.

Die vorliegende Erfindung wird anhand der nachfolgenden Beispiele näher erläutert, diese schränken die Er-findung jedoch in keiner Weise ein. Beispiele, die nicht in den Schutzumfang der Ansprüche fallen, sind nicht Gegenstand der Erfindung.

### Beispiele

Beispiel 1: Imprägnierung von Filterhülsen mit Triton^{®} X-114, Benetzung unmittelbar vor Versuchsbeginn, keine Trocknung.

Zellanzucht und Lyse: E. coli Stamm DH5a mit Plasmid pcDNA 3.1 werden gemäß guter mikrobiologischer Praxis angezogen. Die Zellen werden anschließend durch Zentrifugation geerntet. Das Zellpellet mit ODV = 4850 (ODV = OD600 x Vol [ml]) wird in 130 ml Resuspensionspuffer aufgenommen (50 mM Tris, 10 mM EDTA, pH 8.0 mit 60 µg/ml RNase A). Zur Lyse werden 130 ml Lysepuffer (200 mM NaOH, 1% SDS) zugegeben. Der Ansatz wird durch 15maliges Invertieren gemischt und für 5 min bei Raumtemperatur inkubiert. Anschließend wird der Ansatz mit 130 ml Neutralisa-tionspuffer (3 M Kaliumacetat) versetzt. Der Ansatz wird durch Invertieren (30x) gemischt.

Vorbereitung der Anionenaustauscher: Der hier verwendete Anionenaustauscher der Ansätze A-G besteht aus porösen Silicapartikeln, die mit Methyl-Aminoethanol (MAE) funktionalisiert sind. Von dem pulverförmigen Trennmaterial sind im Midi-Format ca. 600 mg zwischen zwei Polyethylenfritten in der Trennsäule fixiert. Der Anionenaustauscher wird mit 6 ml Äqulibrierungspuffer (100 mM Tris, 15% Ethanol, 900 mM KCl, 0,15% Triton^{®} X-100, pH 6,3) äquilibriert.

Vorbereitung der Filterhülsen: Filterhülsen zur Lysatklärung (gemäß Figur 1, Cellulose, Länge ca. 9 cm, Innendurchmesser ca. 18 mm, Wandstärke ca. 2-3 mm) werden in einer Halterung fixiert und mit je 10 ml folgender Lösungen benetzt: Ansatz A: H₂O; Ansatz B: 0,1% Triton^{®} X-114 in Wasser; Ansatz C: 0,5% Triton^{®} X-114 in Wasser; Ansatz D: 1,0% Triton^{®} X-114 in Wasser; Ansatz E: 5,0% Triton X-114 in Wasser; Ansatz F: 10% Triton^{®} X-114 in Wasser; Ansatz G: Kommerzieller Kit NucleoBond^{®} Xtra Midi EF.

Die Filterhülsen der werden nach Benetzung nicht getrocknet, sondern in feuchtem Zustand in die äquilibrierten Trennvorrichtungen überführt/eingesteckt.

Mit den Ansätzen A-F wird das erfindungsgemäße Verfahren dargestellt. Der Ansatz G wurde mit einem kommerziellen Kit zur Plasmidisolierung (MACHEREY-NAGEL, NucleoBond^{®} Xtra Midi EF, REF 740420) als Referenz durchgeführt. Der Kit ist ein Spezialkit zur Endotoxinabreicherung und liefert transfection grade Plasmid DNA. Dieser Kit verwendet Filterhülsen gemäß Figur 1, die Filterhülsen werden in Ansatz G ohne vorherige Benetzung und/oder Imprägnierung gemäß Standardprotokoll verwendet. Die Verwendung des kommerziellen Produktes soll helfen, die mit dem erfindungsgemäßen Verfahren erzielten Ergebnisse bezüglich des Endotoxingehaltes einzuordnen.

Versuchsdurchführung für Säulen der Ansätze A-F: Vom Lysat werden 23 ml auf die Filter geladen. Das Lysat wird dabei kontinuierlich geschwenkt, damit sich das Präzipitat nicht absetzt. Das Laden der Anionenaustauschersäulen und alle Waschschritte werden mittels gravity flow durchgeführt. Waschen der Säulen mit 5 ml Äquilibrierungspuffer (Puffer wird über die Filterhülse gegeben). Die Filterhülse wird anschließend aus der Trennvorrichtung entnommen und verworfen. Waschen der Säulen mit 8 ml Waschpuffer (100 mM Tris, 15% Ethanol, 1,1 M KCl, pH 6,3). Elution der Plasmide mit 5 ml Elutionspuffer (100 mM Tris, 1,2 M KCl, 15% Ethanol, pH 9,0).

Versuchsdurchführung für Säule G, Ansatz G: Die Durchführung erfolgt nach Standardprotokoll des kommerziellen Kits NucleoBond^{®} Xtra Midi EF. Der Kit verwendet vergleichbare Cellulosefilter für die Lysatklärung, gleiches Anionenaustauschermaterial und eine vergleichbare Pufferchemie. Vom Lysat werden auch hier 23 ml auf die Filter geladen.

Auswertung: Fällung der Plasmid-DNA mit 0,7 Volumenteile Isopropanol (Vortexen, Zentrifugation, 5.000 x g, 15 min, RT, Überstand verwerfen). Das Pellet wird mit 5 ml 70% Ethanol gewaschen (Zentrifugation, 5.000 x g, 5 min, RT, Überstand verwerfen) und in 1000 la.1 Endotoxin-freiem Wasser resuspendiert. Photometrische Vermessung von 10 µl resuspendierter DNA. Endotoxinbestimmung mittels LAL (Limulus Amoebozyten Lysat)-Test: 50 µl Eluat mit 50 µl LAL-Pyrochrom versetzen, das Eluat dabei so verdünnen, dass die die Messwerte innerhalb der Standardgerade liegen (Associates of Cape Cod, Pyrochrome, #C1500).

Ergebnisse: In der folgenden Tabelle sind die DNA Ausbeuten (berechnet über E260) sowie die Reinheiten (als E260/230 und E260/280) dargestellt. Einzelwerte und Mittelwerte von Doppelbestimmungen (fett und kursiv).

**Tabelle 1: DNA Ausbeuten als µg DNA pro Eluat und DNA-Reinheit als E260/230 und E260/280 Ratios (Mittelwerte kursiv).**

| µg DNA/ Eluat | | | | | | |
|---|---|---|---|---|---|---|
| Ansatz A | Ansatz B | Ansatz C | Ansatz D | Ansatz E | Ansatz F | Ansatz G |
| 211 | 229 | 224 | 218 | 251 | 237 | 196 |
| 207 | 204 | 221 | 222 | 239 | 214 | 215 |
| *209* | *217* | *222* | *220* | *245* | *226* | *206* |
| | | | | | | |

| E260/230 | | | | | | |
|---|---|---|---|---|---|---|
| Ansatz A | Ansatz B | Ansatz C | Ansatz D | Ansatz E | Ansatz F | Ansatz G |
| 2,17 | 2,20 | 2,18 | 2,29 | 2,20 | 2,29 | 2,31 |
| 2,20 | 2,18 | 2,22 | 2,22 | 2,26 | 2,28 | 2,32 |
| 2,19 | 2,19 | *2,20* | 2,26 | 2,23 | 2,29 | *2,32* |
| | | | | | | |

| E260/280 | | | | | | |
|---|---|---|---|---|---|---|
| Ansatz A | Ansatz B | Ansatz C | Ansatz D | Ansatz E | Ansatz F | Ansatz G |
| 1,86 | 1,87 | 1,86 | 1,89 | 1,86 | 1,89 | 1,89 |
| 1,87 | 1,85 | 1,86 | 1,86 | 1,89 | 1,89 | 1,90 |
| 1,87 | 1,86 | 1,86 | 1,88 | *1,88* | 1,89 | 1,90 |

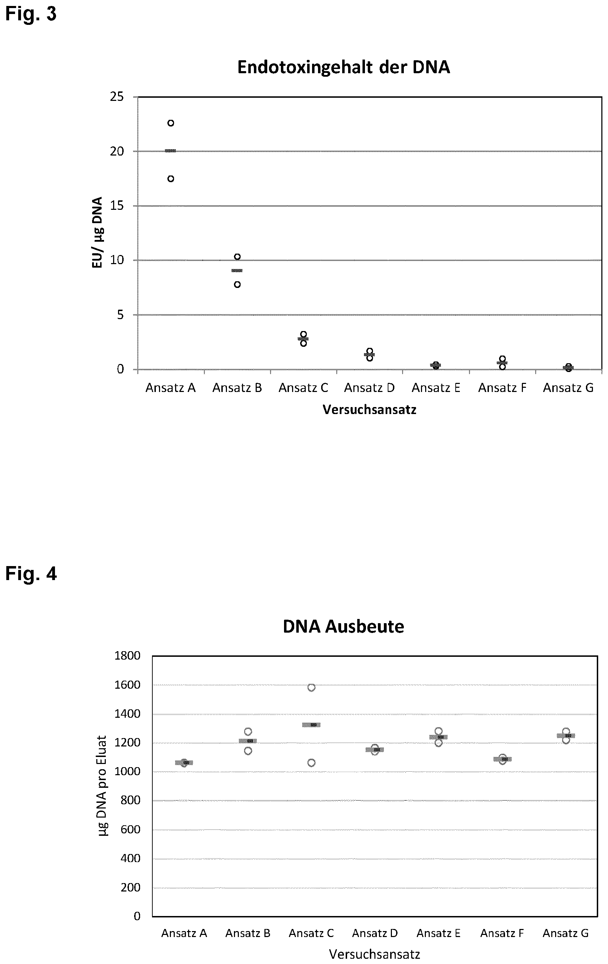

Die DNA Ausbeute ist in Figur 2 dargestellt, die Ergebnisse der Endotoxinbestimmung mittels LAL-Test sind in der Figur 3 gezeigt.

Die Plasmidausbeute ist in allen Ansätzen sehr vergleichbar und liegt etwa zwischen 200 und 230 µg Plasmid DNA (Tabelle 1 und Figur 2). In der Figur 3 ist zu sehen, dass das Detergens Triton^{®} X-114 einen erheblichen Einfluss auf den Endotoxingehalt der DNA hat. Durch Benetzung der Filterhülsen mit Triton^{®} X-114 kann das Endotoxinniveau der Eluate deutlich reduziert werden. Je mehr Triton vorhanden ist, desto weniger Endotoxine werden in den Eluaten gemessen. Gegenüber dem Filter ohne Triton^{®} X-114 sinken die Endotoxinwerte der mit der 5% Triton^{®} X-114 Lösung imprägnierten Filter um ca. 98%. Damit werden Endotoxingehalte von < 1 EU/µg DNA erhalten. DNA Ausbeute und DNA-Reinheit werden durch die Imprägnierung der Filter mit Triton^{®} X-114 nicht beeinträchtigt (Figur 1). Erwartungsgemäß liefert auch der kommerziell erhältliche Spezialkit zur Endotoxinabreicherung sehr niedrige Endotoxinwerte. Bei diesem Verfahren werden die Endotoxine mit einem Triton^{®} X-114-haltigem Äquilibrierungs- und Waschpuffer entfernt. Diese Daten dienen nur zur größenmäßigen Einordnung der Ergebnisse nach dem erfindungsgemäßen Verfahren.

Beispiel 2: Imprägnierung von Filterhülsen mit Triton^{®} X-114, Trocknung der Filter, Äquilibrierung nur der Anionenaustauscher.

Zellanzucht und Lyse: Die Bakterienanzucht erfolgte wie im Beispiel 1. Abweichend wurde ein Zellpellet mit ODV 6.000 verwendet, jeweils 120 ml Resuspensionspuffer, Lysepuffer, Neutralisationspuffer. Es werden 24 ml Lysat pro Probe eingesetzt.

Vorbereitung der Filterhülsen: Filterhülsen zur Lysatklärung (gemäß Figur 1) wurden gemäß dem folgenden Schema mit Triton^{®}X-114 imprägniert und vor dem Versuch vollständig getrocknet (10 ml, Raumtemperatur, 4 Wochen).
- Ansatz A:: Verwendung einer trockenen, nicht zuvor imprägnierten Filterhülse.
- Ansatz B:: Filterhülse nur mit Wasser benetzt und getrocknet.
- Ansatz C:: Filterhülse mit 2,5% Triton^{®} X-114 benetzt und getrocknet.
- Ansatz D:: Filterhülse mit 5,0% Triton^{®} X-114 benetzt und getrocknet
- Ansatz E:: Filterhülse mit 7,5% Triton^{®} X-114 benetzt und getrocknet
- Ansatz F:: Filterhülse mit 10,0% Triton^{®} X-114 benetzt und getrocknet
- Ansatz G:: Kommerzieller Kit NucleoBond^{®} Xtra Midi Plus

Der Ansatz G wurde mit einem kommerziellen Kit zur Plasmidisolierung (MACHEREY-NAGEL, NucleoBond^{®} Xtra Midi Plus, REF 740412.50) als Referenz durchgeführt. Der Kit ist ein Anionenaustauscherkit ohne besondere Maßnahmen zur Endotoxinabreicherung. Dieser Kit verwendet Filterhülsen gemäß Figur 1, die Filterhülsen werden in Ansatz G ohne vorherige Benetzung und/oder Imprägnierung gemäß Standardprotokoll verwendet. Die Verwendung des kommerziellen Produktes soll helfen, die mit dem erfindungsgemäßen Verfahren erzielten Ergebnisse bezüglich des Endotoxingehaltes einzuordnen. Versuchsdurchführung für Säulen der Ansätze A-F: Die Durchführung der Plasmidisolierung und die Auswertung erfolgte wie im Beispiel 1 beschrieben. In den Ansätzen A-F wurde der Anionenaustauscher mit 12 ml Äquilibrierungspuffer äquilibriert, anschließend wurden die Filterhülsen in die Trennvorrichtungen überführt/eingesteckt. Die Durchführung der Ansätze A-F gemäß Beispiel 1 (Versuchsdurchführung für Säulen A-F), Durchführung des Ansatzes G nach Standardprotokoll des kommerziellen Kits NucleoBond^{®} Xtra Midi. Der Kit verwendet vergleichbare Cellulosefilter für die Lyatklärung, gleiches Anionenaustauschermaterial und eine vergleichbare Pufferchemie. Vom Lysat werden auch hier 24 m auf die Filter geladen.

Ergebnisse: Wie die Figur 4 zeigt, wurden in der DNA-Ausbeute keine wesentlichen Unterschiede zwischen den Ansätzen beobachtet. Mit dem Standardprotokoll des kommerziellen Produktes (Ansatz G) wurden 1,25 mg an Plasmid-DNA als Ausbeute erhalten. Die DNA-Ausbeute bei den Triton^{®} X-114 imprägnierten Filtern (Ansatz C-F) schwankt zwischen 1,32 und 1,08 mg und ist damit sowohl zum Standardprotokoll vergleichbar, als auch zur Kontrolle mit den Filterhülsen ohne Benetzung (Ansatz A) und den mit Wasser-benetzten Filtern (Ansatz B).

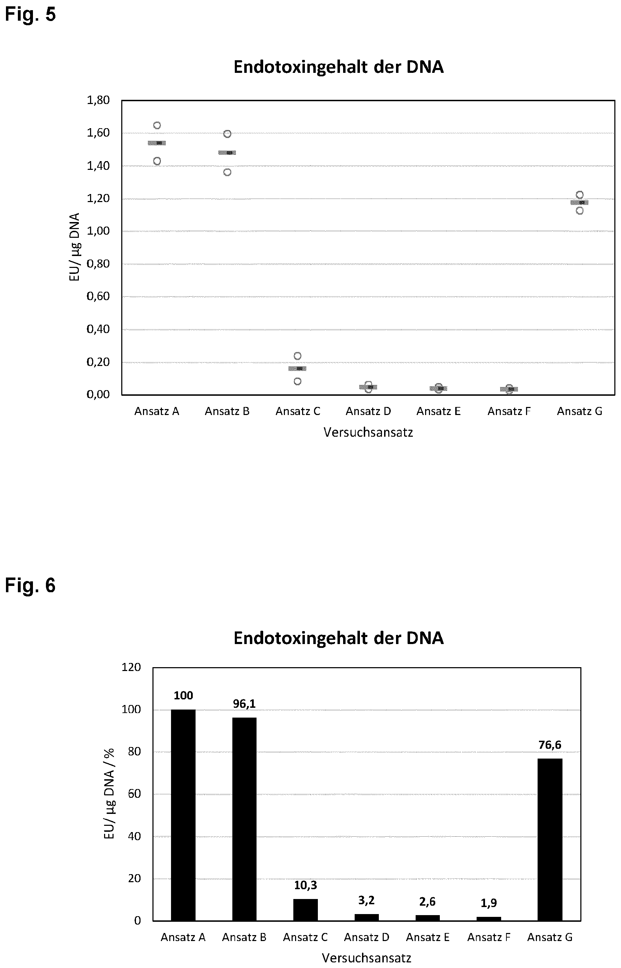

Der Einfluss der Imprägnierung mit Triton^{®} X-114 wird in Figur 5 deutlich. Hier ist der Endotoxingehalt der DNA für die einzelnen Versuchsansätze gezeigt. Die Endotoxine liegen beim Standardverfahren bei 1,18 EU pro µg DNA (Ansatz G). Dies entspricht den Erwartungen für ein Protokoll ohne besondere Endotoxinabreicherung. Vergleichbare Ergebnisse wurden in den Kontrollen (Ansätze A und B) mit Filterhülsen ohne Triton^{®} X-114 erhalten. Die Imprägnierung der Filterhülsen mit Triton^{®} X-114 führt hingegen zu einer deutlichen Reduktion der Endotoxine. Bereits die Imprägnierung mit einer 2,5%igen Triton^{®} X-114 Lösung (Ansatz C) führt bezogen auf die Kontrolle in Ansatz A zu einer Reduktion der Endotoxine um ca. 90% (prozentuale Darstellung in der Figur 6). Filterhülsen, die mit einer 10%igen Triton^{®} X-114 Lösung imprägniert wurden (Ansatz F), führten zu Endotoxingehalten von lediglich 0,03 EU/µg DNA. Dies entspricht gerade einmal 2 % des Kontrollansatzes A. Die Verwendung imprägnierter Filterhülsen reduziert die Endotoxine also um ca. 98 % so dass Werte erhalten werden, die im Bereich kommerzieller Spezialprodukte für die Endotoxinabreicherung liegen.

Die Verwendung imprägnierter und getrockneter Filterhülsen führt hier zu einer Möglichkeit, gänzlich auf Triton^{®} X-114 in Äquilibrierungs- und Waschpuffern zu verzichten.

Beispiel 3: Imprägnierung von Filterhülsen mit Triton^{®} X-114, Trocknung der Filter, Äquilibrierung der Anionenaustauscher über die eingesteckten Filterhülsen.

Zellanzucht und Lyse: Die Bakterienanzucht erfolgte wie im Beispiel 1. Abweichend wurde ein Zellpellet mit ODV 3600 verwendet, jeweils 72 ml Resuspensionspuffer, Lysepuffer, Neutralisationspuffer. Es werden 24 ml Lysat pro Probe eingesetzt. Das Masterlysat wird zu gleichen Teilen auf 3 Ansätze (je 3-fach) verteilt.

### Vorbereitung der Filterhülsen:

Ansatz A: Verwendung einer trockenen, nicht zuvor imprägnierten Filterhülse. Äquilibrieren des Anionenaustauschers mit eingesteckter Filterhülse (Auftrag der Äqulibrierungslösung auf den Cellulosefilter, Lösung läuft durch den Filter und benetzt anschließend den darunterliegenden Anionenaustauscher).

Ansatz B: Filterhülse mit 10 ml einer 10% Triton^{®} X-114 imprägniert und getrocknet (60 h, RT). Äquilibrieren des Anionenaustauschers ohne eingesteckte Filterhülse. Auftrag der Äqulibrierungslösung nur auf den Anionenaustauscher, anschließend wird die trockene Filterhülse in die Trennvorrichtung eingesteckt. Ansatz C: Filterhülse mit 10 ml einer 10% Triton^{®} X-114 imprägniert und getrocknet (60 h, RT). Äquilibrieren des Anionenaustauschers mit eingesteckter Filterhülse (Auftrag der Äqulibrierungslösung auf den Cellulosefilter, Lösung läuft durch den Filter und benetzt anschließend den darunterliegenden Anionenaustauscher).

Versuchsdurchführung: Nach Äquilibrierung werden 24 ml Masterlysat auf jeden Filter aufgetragen. Waschen mit 5 ml Äquilibrierungspuffer. Entnahme der Filterhülsen aus dem Säulenkörper (verwerfen). Waschen der Trennsäule mit 8 ml Waschpuffer 1, Elution der Plasmid-DNA mit 5 ml Elutionspuffer 1. Anschließend DNA-Fällung und Auswertung wie in Beispiel 1 beschrieben.

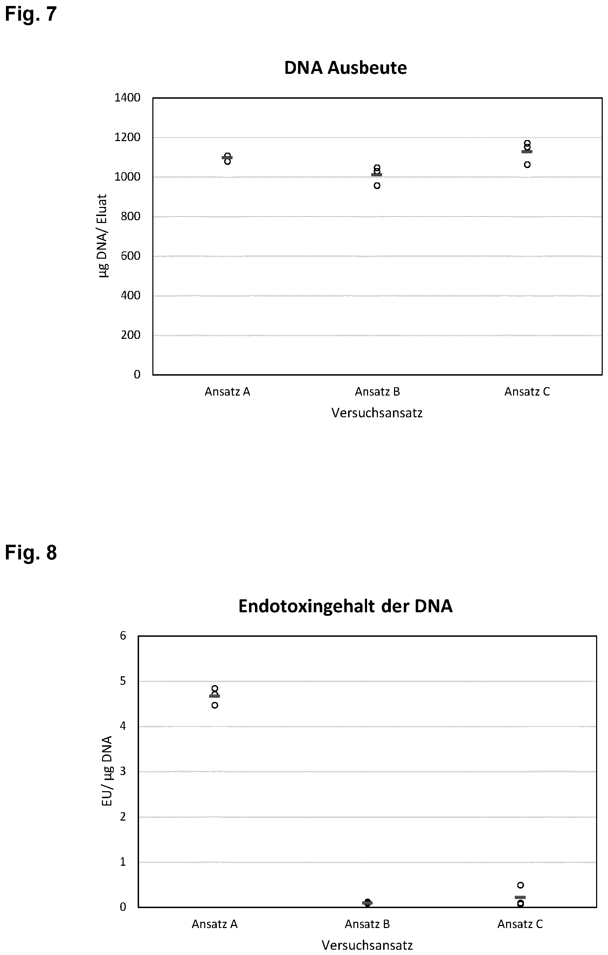

Ergebnisse: Wie die Figur 7 zeigt, wurden in der DNA-Ausbeute keine wesentlichen Unterschiede zwischen den Ansätzen beobachtet. In allen Fällen lag die Ausbeute bei ca. 1000 bis 1100 µg DNA pro Eluat. Zudem ist die Reproduzierbarkeit sehr gut, die Schwankungen der Einzelwerte sind gering. Auch die Reinheiten der Plasmid-DNA sind sehr einheitlich. Für die Ansätze A-C wurden E260/230 Ratios von 2,31, 2,32 und 2,31 erhalten. Die Ratios E260/280 liegen bei 1,89, 1,89 und 1,88. Auf einem Agarosegel (Daten hier nicht gezeigt) zeigt sich die DNA als homogene Bande ohne Degradierung oder RNA-Kontamination.

Die Ergebnisse der Endotoxinbestimmung sind in Figur 8 gezeigt. Im Ansatz A ohne die Vorbehandlung der Filterhülsen mit Triton^{®} X-114 finden sich im Mittel 4,7 EU/µg DNA. Dies entspricht der Erwartung für ein Verfahren ohne besondere Maßnahmen zur Endotoxinabreicherung. Im Ansatz B liegt der Endotoxingehalt im Mittel nur bei 0,09 EU/ µg DNA. Damit werden die Ergebnisse aus den vorherigen Versuchen bestätigt; der Einsatz von Triton^{®} X-114 führt zu einer deutlichen Reduzierung der Endotoxine gegenüber den Standardverfahren. Im Ansatz B wurde der Filter jedoch nicht mit Äquilibrierungspuffer benetzt, um das Detergens nicht vorzeitig (d.h. vor dem Kontakt mit dem Bakterienlysat) aus dem Filter auszuwaschen. Überraschenderweise wurde aber mit dem Ansatz C beobachtet, dass die Filterhülse bei der Äquilibrierung in der Trennsäule verbleiben kann. Wird der Äquilibrierungspuffer nicht nur über den Anionenaustauscher, sondern auch über die eingesteckte Filterhülse gegeben, so werden ebenfalls sehr niedrige Endotoxingehalte im Mittel von 0,22 EU/µg DNA erhalten. Offensichtlich wird nur ein Teil des Detergens während der Äquilibrierung ausgewaschen, so dass noch ausreichend Triton^{®} X-114 beim Kontakt mit dem Bakterienlysat zur Verfügung steht. Auch das Auswaschen von Detergens und das Inkontaktbringen des Anionenaustauschers mit dem Detergens während der Äquilibrierung trägt zu diesem überraschenden Effekt bei.

Durch Einsatz der mit Triton^{®} X-114 imprägnierten Filterhülsen kann also der Endotoxingehalt der isolierten DNA erheblich reduziert werden. Es können Endotoxingehalte erreicht werden, die mit herkömmlichen Spezialprodukten zur Endotoxinabreicherung vergleichbar sind. Mit der fabrikationsseitigen Einbringung des Triton^{®} X-114 Detergens auf die Filterhülsen ergibt sich eine Möglichkeit, das Detergens nicht über die üblichen Puffer und Lösungen einzubringen, sondern über die Filterhülsen in den Prozess einzubringen. Damit sind auch Konzepte mit gefriergetrockneten Reagenzien denkbar; das Triton^{®} X-114 wird dabei getrocknet über die imprägnierten Filterhülsen eingebracht.

## Patentansprüche

1. Verfahren zur Plasmidreinigung unter gleichzeitiger Abreicherung von Endotoxinen umfassend die Lysatklärung eines Plasmid-haltigen Zelllysates in einem mit nicht-ionischen Detergens beladenen zylindrischem Tiefenfilter, das direkte Überführen des Filtrats mit dem geklärten Zelllysat auf einen fluidisch nachgeschalteten Anionenaustauscher, und die Elution des gereinigten und Endotoxin-abgereicherten Plasmidmaterials von dem Anionenaustauscher durch einen Elutionspuffer, wobei der mit nicht-ionischen Detergens beladene Tiefenfilter mit dem nicht-ionischen Detergens imprägniert ist oder das nicht-ionische Detergens bei der Tiefenfilterherstellung in das Fasergeflecht eingebracht wird, und wobei der zylindrische Tiefenfilter und der Anionenaustauscher in einer Trennvorrichtung vorliegen, die ein säulenartiges Außengefäß (4) mit einer obenseitigen Einfüllöffnung (3) und mit dem darin eingesetzten zylindrischen Tiefenfilter (1), der eine Filterhülse mit einem Filterboden und einem davon hochgehenden Filtermantel ist, wenigstens eine poröse Trägerschicht (5) und eine auf dieser Trägerschicht (5) befindliche Anionenaustauscherschicht (6) umfasst.

2. Verfahren nach Anspruch 1, wobei das Verfahren die folgenden Schritte umfasst:
(a) Lyse des Zellmaterials,
(b) Überführen des Zelllysates in einen mit nicht-ionischen Detergens beladenen zylindrischen Tiefenfilter,
(c) direktes Überführen des Filtrats mit dem geklärten Zelllysat auf den fluidisch nachgeschalteten Anionenaustauscher,
(d) Waschen des Tiefenfilters und des fluidisch nachgeschalteten Anionenaustauschers mit einem Wasch-/Äquilibrierungspuffer und
(e) Elution des gereinigten Plasmidmaterials von dem Anionenaustauscher durch einen Elutionspuffer,
wobei vorzugsweise das Verfahren die folgenden zwei separaten Waschschritte (d1) und (d2) umfasst:
(d1) das Waschen des Tiefenfilters und des fluidisch nachgeschalteten Anionenaustauschers mit einem ersten Wasch-/Äquilibrierungspuffer und
(d2) das direkte Waschen des Anionenaustauschers mit einem zweiten Waschpuffer.

3. Verfahren nach Anspruche 1 oder 2, wobei der zylindrische Tiefenfilter aus einem regelosen Fasergeflecht, welches
(i) aus Cellulose-, Glas-, Kunststoff- und Metallfasern und Gemischen derselben besteht, wobei Cellulosefasern besonders bevorzugt sind; und/oder
(ii) Eigenstabilität aufweist.

4. Verfahren nach einem oder mehreren der Ansprüche 1 bis 3, wobei das nichtionische Detergens ein Polyethylenglycol-[4-(1,1,3,3-tetramethylbutyl)phenyl]ether, bevorzugt ein Polyethylenglycol-[4-(1,1,3,3-tetramethylbutyl)-phenyl]ether mit 7 bis 8 oder 9 bis 10 Ethylenglycoleinheiten (Triton^{®} X-114 bzw. Triton^{®} X-100) ist.

5. Verfahren nach einem oder mehreren der Ansprüche 1 bis 4, wobei der Anionenaustauscher aus funktionalisierten Silicapartikeln besteht und bevorzugt die Silicapartikel mit Methylaminoethanol oder Diethylaminoethanol funktionalisiert sind.

6. Verfahren nach einem oder mehreren der Ansprüche 1 bis 5, wobei der Elutionspuffer und/oder der Wasch/Äquilibrierungspuffer Detergens-frei sind.

7. Verfahren nach einem oder mehreren der Ansprüche 1 bis 6, wobei die Lyse des Zellmaterials durch Versetzen mit einem alkalischen Lysepuffer und nachfolgender Neutralisation mittels eines Neutralisationspuffers erfolgt, wobei diese Puffer vorzugsweise frei von nicht-ionischen Detergenzien sind.

8. Verfahren nach einem oder mehreren der Ansprüche 1 bis 7, wobei
(i) der Wasch/Äquilibrierungspuffer Tris, Ethanol, ein für den jeweiligen Anionenaustauscher geeignetes Anion und ein nichtionisches Detergens enthält, wobei das nichtionische Detergens, wenn der Anionenaustauscher nicht gemeinsam mit dem Tiefenfilter in einer Trennvorrichtung vorliegt, nicht nur ein Polyethylenglycol-[4-(1,1,3,3-tetramethylbutyl)phenyl]ether wie Triton^{®} X-114 und Triton^{®} X-100, sondern auch ein ethoxylierter Sorbitanfettsäureester wie Tween^{®} 20 (Polyoxyethylen(20)-sorbitan-monolaurat) oder ein anderes nichtionisches Detergens sein kann; und/oder
(ii) das Volumen des Wasch/Äquilibrierungspuffers im Verhältnis von 10 bis 20 zum Totvolumen des Anionenaustauschers steht.

9. Verfahren nach einem oder mehreren der Ansprüche 1 bis 8, wobei
(i) der Elutionspuffer Salze mit sterisch kleinen Anionen als Gegenionen für den Anionenaustauscher, insbesondere Salze mit Chloridionen enthält; und/oder
(ii) die Elution durch einen pH-Shift in den alkalischen pH Bereich in Bezug auf den pH des Wasch/Äquilibrierungspuffers unterstützt wird; und/oder
(iii) das Volumen des Elutionspuffers im Verhältnis von 5 bis 20 zum Totvolumen des Anionenaustauschers steht.

10. Mit nicht-ionischen Detergens beladener zylindrischer Tiefenfilter wie in Ansprüchen 1 bis 4 definiert.

11. Trennvorrichtung wie in den Ansprüchen 1 und 3 bis 5 definiert mit einem wie in Ansprüchen 1 bis 4 definierten, mit nicht-ionischen Detergens beladenen, zylindrischen Tiefenfilter.

12. Kit zur Plasmidreinigung unter gleichzeitiger Abreicherung von Endotoxinen umfassend wenigstens einen mit nicht-ionischen Detergens beladenen zylindrischen Tiefenfilter nach Anspruch 10 und/oder wenigstens eine Trennvorrichtung nach Anspruch 11.

13. Kit nach Anspruch 12, weiterhin umfassend einen oder mehrere geeigneter Lysepuffer, Neutralisationspuffer, Wasch-/Äquilibrierungspuffer und Elutionspuffer, insbesondere wie in Ansprüchen 6 bis 9 definiert.

14. Kit nach Anspruch 13, wobei einer oder mehrere der Puffer Detergens-frei sind und als Lyophilisate in dem Kit vorliegen.

## Claims

1. A process for plasmid purification with simultaneous reduction of endotoxin levels, comprising lysate clarification of a cell lysate containing plasmids in a cylindrical deep filter loaded with a non-ionic surfactant, direct transfer of the filtrate with the cleared cell lysate to a downstream anion exchanger, and elution of the purified and endotoxin-reduced plasmid material from the anion exchanger by using an elution buffer, wherein said deep filter loaded with the non-ionic surfactant is impregnated with said non-ionic surfactant, or said non-ionic surfactant is incorporated into said fibrous fabric during the production of the deep filter, and wherein said cylindrical deep filter and said anion exchanger are provided in a separation device comprising a columnar outer vessel (4) with a top side filling hole (3) and a cylindrical deep filter (1) inserted therein, which is a filter sleeve with a filter bottom and a filter coat rising up therefrom, at least one porous support layer (5), and an anion exchanger layer (6) provided on said support layer (5).

2. The process according to claim 1, wherein said process comprises the following steps:
(a) lysis of the cell material,
(b) transfer of the cell lysate to a cylindrical deep filter loaded with a non-ionic surfactant,
(c) direct transfer of the filtrate with the cleared cell lysate to said downstream anion exchanger,
(d) washing the deep filter and the downstream anion exchanger with a washing/equilibration buffer, and
(e) elution of the purified plasmid material from the anion exchanger by using an elution buffer,
wherein preferably said process comprises the following two separate washing steps (d1) and (d2):
(d1) washing the deep filter and the downstream anion exchanger with a first washing/equilibration buffer, and
(d2) directly washing the anion exchanger with a second washing buffer.

3. The process according to claims 1 or 2, wherein said cylindrical deep filter consists of a random fibrous fabric, which
(i) consists of cellulose, glass, plastic and metallic fibers, and mixtures thereof, wherein cellulose fibers are particularly preferred; and/or
(ii) has intrinsic stability.

4. The process according to one or more of claims 1 to 3, wherein said non-ionic surfactant is a polyethylene glycol [4-(1,1,3,3-tetramethylbutyl)-phenyl] ether, more preferably a polyethylene glycol [4-(1,1,3,3-tetramethylbutyl)-phenyl] ether with 7 to 8 or 9 to 10 ethylene glycol moieties (Triton^{®} X-114 or Triton^{®} X-100).

5. The process according to one or more of claims 1 to 4, wherein said anion exchanger consists of functionalized silica particles, and said silica particles are preferably functionalized with methylaminoethanol or diethylaminoethanol.

6. The process according to one or more of claims 1 to 5, wherein said elution buffer and/or said washing/equilibration buffer are surfactant-free.

7. The process according to one or more of claims 1 to 6, wherein said lysis of the cell material is effected by admixing with an alkaline lysis buffer, followed by neutralization by means of a neutralization buffer, wherein said buffers are preferably free of non-ionic surfactants.

8. The process according to one or more of claims 1 to 7, wherein
(i) said washing/equilibration buffer contains Tris, ethanol, an anion suitable for the respective anion exchanger, and a non-ionic surfactant, wherein, when said anion exchanger is not provided together with the deep filter in a separation device, said non-ionic surfactant may be not only a polyethylene glycol [4-(1,1,3,3-tetramethylbutyl)phenyl] ether, such as Triton^{®} X-114 and Triton^{®} X-100, but may also be an ethoxylated sorbitan fatty acid ester, such as Tween 20 (polyoxyethylene(20)sorbitan monolaurate), or some other non-ionic surfactant; and/or
(ii) the ratio of the volume of said washing/equlibration buffer to the dead volume of the anion exchanger is from 10 to 20.

9. The process according to one or more of claims 1 to 8, wherein
(i) said elution buffer contains salts with sterically small anions as counterions for the anion exchanger, especially salts with chloride ions; and/or
(ii) the elution is promoted by a pH shift into an alkaline pH range as compared to the pH of the washing/equlibration buffer; and/or
(iii) the ratio of the volume of said elution buffer to the dead volume of the anion exchanger is from 5 to 20.

10. A cylindrical deep filter loaded with a non-ionic surfactant as defined in claims 1 to 4.

11. A separation device as defined in claims 1 and 3 to 5 comprising a cylindrical deep filter loaded with a non-ionic surfactant as defined in claims 1 to 4.

12. A kit for plasmid purification with simultaneous reduction of endotoxin levels, comprising at least one cylindrical deep filter loaded with a non-ionic surfactant according to claim 10; and/or at least one separation device according to claim 11.

13. The kit according to claim 12, further comprising one or more suitable lysis buffers, neutralization buffers, washing/equlibration buffers, and elution buffers, especially as defined in claims 6 to 9.

14. The kit according to claim 13, wherein one or more of said buffers are surfactant-free and are provided as lyophilizates in said kit.

## Revendications

1. Procédé de purification de plasmides avec appauvrissement simultané d'endotoxines, comprenant la clarification d'un lysat cellulaire contenant un plasmide dans un filtre en profondeur cylindrique chargé en détergent non ionique, le transfert direct du filtrat avec le lysat cellulaire clarifié vers un échangeur d'anions fluidiquement monté en aval, et l'élution du matériel plasmidique purifié et appauvri en endotoxines, par l'échangeur d'anions grâce à un tampon d'élution, dans lequel le filtre en profondeur chargé en détergent non ionique est imprégné du détergent non ionique, ou dans lequel le détergent non ionique est introduit dans le treillis de fibres lors de la fabrication du filtre en profondeur, et dans lequel le filtre en profondeur cylindrique et l'échangeur d'anions se trouvent dans un dispositif de séparation comprenant un récipient extérieur (4) de type colonne avec un orifice de remplissage (3) situé côté supérieur et avec le filtre en profondeur cylindrique (1) inséré dans ledit orifice, et comportant un fond de filtre et une enveloppe de filtre s'élevant à partir dudit fond de filtre, au moins une couche de support poreuse (5) et une couche d'échangeur d'anions située sur ladite couche de support (5).

2. Procédé selon la revendication 1, dans lequel le procédé comprend les étapes ci-dessous :
(a) lyse du matériel cellulaire ;
(b) transfert du lysat cellulaire dans un filtre en profondeur cylindrique chargé de détergent non ionique,
(c) transfert direct du filtrat avec le lysat cellulaire clarifié vers l'échangeur d'anions fluidiquement monté en aval,
(d) lavage du filtre en profondeur et de l'échangeur d'anions fluidiquement monté en aval avec un tampon de lavage/d'équilibrage, et
(e) élution, par l'échangeur d'anions et grâce à un tampon d'élution, du matériel plasmidique purifié,
le procédé comprenant de manière préférée les deux étapes de lavage séparées (d1) et (d2) ci-dessous :
(d1) lavage, avec un premier tampon de lavage/d'équilibrage, du filtre en profondeur et de l'échangeur d'anions fluidiquement monté en aval, et
(d2) lavage direct de l'échangeur d'anions avec un second tampon de lavage.

3. Procédé selon la revendication 1 ou 2, dans lequel le filtre en profondeur cylindrique est constitué d'un treillis irrégulier de fibres qui
(i) est constitué de fibres de cellulose, de verre, de plastique et de métal et de mélanges de celles-ci, les fibres de cellulose étant particulièrement préférées ; et/ou
(ii) présente une stabilité intrinsèque.

4. Procédé selon une ou plusieurs des revendications 1 à 3, le détergent non ionique étant un [4-(1,1,3,3-tétraméthylbutyl)phényl]éther de polyéthylèneglycol, de préférence un [4-(1,1,3,3-tétraméthylbutyl)phényl]éther de polyéthylèneglycol avec 7 à 8 ou 9 à 10 unités éthylèneglycol (Triton^{®} X-114 ou Triton^{®} X-100).

5. Procédé selon l'une quelconque ou plusieurs des revendications 1 à 4, l'échangeur d'anions étant constitué de particules de silice fonctionnalisées et les particules de silice étant de manière préférée fonctionnalisées avec du méthylaminoéthanol ou du diéthylaminoéthanol.

6. Procédé selon l'une quelconque ou plusieurs des revendications 1 à 5, le tampon d'élution et/ou le tampon de lavage/d'équilibrage étant exempts de détergent.

7. Procédé selon l'une quelconque ou plusieurs des revendications 1 à 6, la lyse du matériel cellulaire étant mise en œuvre par mélange avec un tampon de lyse alcalin et neutralisation subséquente au moyen d'un tampon de neutralisation, lesdits tampons étant de manière préférée exempts de détergents non ioniques.

8. Procédé selon l'une quelconque ou plusieurs des revendications 1 à 7,
(i) le tampon de lavage/d'équilibrage contenant du tris, de l'éthanol, un anion approprié pour l'échangeur d'anions respectif et un détergent non ionique, si l'échangeur d'anions ne se trouve pas dans un dispositif de séparation en même temps que le filtre en profondeur, le détergent non ionique pouvant non seulement être un [4-(1,1,3,3-tétraméthylbutyl)phényl]éther de polyéthylèneglycol tel que le Triton^{®} X-114 et le Triton^{®} X-100, mais pouvant aussi être un ester d'acide gras de sorbitane éthoxylé tel que le Tween^{®} 20 (monolaurate de polyoxyéthylène(20)-sorbitane) ou un autre détergent non ionique ; et/ou
(ii) le volume du tampon de lavage/d'équilibrage se situant dans un rapport de 10 à 20 par rapport au volume mort de l'échangeur d'anions.

9. Procédé selon l'une quelconque ou plusieurs des revendications 1 à 8,
(i) le tampon d'élution contenant des sels avec des anions stériquement petits faisant office de contre-ions pour l'échangeur d'anions, en particulier des sels avec des ions chlorure ; et/ou
(ii) l'élution étant favorisée par un décalage du pH dans la plage de pH alcalin par rapport au pH du tampon de lavage/d'équilibrage ; et/ou
(iii) le volume du tampon d'élution se situant dans un rapport de 5 à 20 par rapport au volume mort de l'échangeur d'anions.

10. Filtre en profondeur cylindrique chargé de détergent non ionique tel que défini dans les revendications 1 à 4.

11. Dispositif de séparation tel que défini dans les revendications 1 et 3 à 5, comprenant un filtre en profondeur cylindrique chargé de détergent non ionique tel que défini dans les revendications 1 à 4.

12. Kit de purification de plasmides avec appauvrissement simultané d'endotoxines, comprenant au moins un filtre en profondeur cylindrique chargé en détergent non ionique selon la revendication 10 et/ou au moins un dispositif de séparation selon la revendication 11.

13. Kit selon la revendication 12, comprenant en outre un ou plusieurs tampon(s) de lyse, tampon(s) de neutralisation, tampon(s) de lavage/d'équilibrage et tampon(s) d'élution approprié(s), en particulier tel que défini dans les revendications 6 à 9.

14. Kit selon la revendication 13, un ou plusieurs des tampons étant exempts de détergent et se trouvant dans le kit sous forme de lyophilisats.
